# EUROPEAN PATENT APPLICATION

(11) **EP 4 029 808 A1**
(43) Date of publication of application: **20.07.2022**
(21) Application number: 21863887.2
(22) Date of filing: 27.04.2021
(51) Int. Cl.: B65D 83/14, B65D 83/54

(54) **CONTINUOUS FLOW VALVE, HIGH-PRESSURE CONTAINER, AND ASTHMA TREATMENT DEVICE**

(30) Priority: 04.09.2020 JP 2020149493
(71) Applicant: Pacific Industrial Co., Ltd., Ogaki-shi, Gifu 503-8603 (JP)
(72) Inventor: KANAMORI, Kazuhiro, Ogaki-shi, Gifu 503-8603 (JP)
(74) Representative: Kramer Barske Schmidtchen Patentanwälte PartG mbB
(86) International application number: PCT/JP2021/016852
(87) International publication number: WO 2022/049828

(57) **Abstract**

[Problem] Provided is a metering valve capable of improving stability of guiding and supporting a stem when a metered quantity of fluid is ejected.

[Solution] A metering valve 10 includes: a high-pressure partition wall 93 that forms part of a high-pressure container 90 that isolates a storage region R1 to be filled with a high-pressure fluid from an atmospheric pressure space; an internal partition wall 33 that is arranged inside the high-pressure partition wall 93 and forms a metering chamber R2 partitioned from the storage region R1 between the internal partition wall and the high-pressure partition wall 93; a stem 20 that extends in a direction in which the high-pressure partition wall 93 and the internal partition wall 33 face each other, is linearly movably supported, and is biased toward the high-pressure partition wall 93 to be positioned at an origin position; a first seal hole 31 that penetrates the high-pressure partition wall 93 so as to extend over the atmospheric pressure space and the metering chamber R2 and has a first seal portion 31S linearly movably supporting a first intermediate portion 21 of the stem 20 and closing a gap between the first seal hole 31 and the stem 20; a second seal hole 32 that penetrates the internal partition wall 33 so as to extend over the metering chamber R2 and the storage region R1 and has a second seal portion linearly movably supporting a second intermediate portion 22 of the stem 20 and closing a gap between the second seal hole 32 and the stem 20; and a starting-end-side stem internal flow path R4 that is formed inside the stem 20 and extends from a starting end of the stem 20 to the second intermediate portion 22, the starting end being always positioned in the storage region R1.

## Description

### TECHNICAL FIELD

The present disclosure relates to a metering valve that allows a specific amount of fluid to flow out from a container at a time and a high-pressure container and an asthma treatment device each of which includes the metering valve.

### BACKGROUND ART

There is known a conventional metering valve that temporarily stores a high-pressure fluid from a fluid storage region in a metering chamber inside a container and ejects the fluid from the metering chamber into an atmospheric pressure space while blocking the metering chamber and the fluid storage region (see, for example, Patent Document 1). In this metering valve, a stem penetrating the metering chamber and having a fluid ejection port at a distal end thereof axially moves and is arranged at a metering chamber filling position where the metering chamber is filled with the fluid from the fluid storage region and a metered quantity discharge position where the fluid is ejected from the metering chamber into the atmospheric pressure space, and thus the metering valve is opened and closed.

### RELATED ART DOCUMENTS

### PATENT DOCUMENTS

Patent Document 1: JP 2003-267462 A (Figs. 1 to 8 and the like)

### SUMMARY OF THE INVENTION

### PROBLEMS TO BE SOLVED BY THE INVENTION

A conventional metering valve includes a stem guide portion to which a base end portion of the stem is fitted and capable of guiding linear movement of the stem, and the metering chamber is filled with a fluid by allowing the fluid to pass through a gap between the stem guide portion and the stem. The stem guide portion is spaced apart from the stem as described above, which causes a problem that stability of guiding the stem from the metering chamber filling position to the metered quantity discharge position and supporting the stem is low.

### MEANS OF SOLVING THE PROBLEMS

A metering valve according to one aspect of the invention that has been made to solve the above problems includes: a high-pressure partition wall that forms part of a high-pressure container that isolates a storage region to be filled with a high-pressure fluid from an atmospheric pressure space; an internal partition wall that is arranged inside the high-pressure partition wall and forms a metering chamber partitioned from the storage region between the internal partition wall and the high-pressure partition wall; a stem that extends in a direction in which the high-pressure partition wall and the internal partition wall face each other, is linearly movably supported, and is biased toward the high-pressure partition wall to be positioned at an origin position; a first seal hole that penetrates the high-pressure partition wall so as to extend over the atmospheric pressure space and the metering chamber and has a first seal portion linearly movably supporting a first intermediate portion of the stem and closing a gap between the first seal hole and the stem; a second seal hole that penetrates the internal partition wall so as to extend over the metering chamber and the storage region and has a second seal portion linearly movably supporting a second intermediate portion of the stem and closing a gap between the second seal hole and the stem; a terminal-end-side stem internal flow path that is formed inside the stem and extends from a terminal end of the stem to the first intermediate portion, the terminal end being always positioned in the atmospheric pressure space; a starting-end-side stem internal flow path that is formed inside the stem and extends from a starting end of the stem to the second intermediate portion, the starting end being always positioned in the storage region; a terminal end discharge port that is provided in the terminal-end-side stem internal flow path and is open to the terminal end of the stem; a peripheral surface inlet port that is provided in the terminal-end-side stem internal flow path, is open to an outer peripheral surface of the first intermediate portion of the stem, is positioned to be closer to the atmospheric pressure space than to the metering chamber when the stem is arranged at the origin position, and is positioned in the metering chamber when the stem is moved to a metered quantity discharge position from the origin position; a starting end inlet port that is provided in the starting-end-side stem internal flow path and is open to the starting end of the stem; and a peripheral surface discharge port that is provided in the starting-end-side stem internal flow path, is open to an outer peripheral surface of the second intermediate portion of the stem, is positioned in the metering chamber when the stem is arranged at the origin position, moves from the metering chamber to the storage region before the peripheral surface inlet port is positioned in the metering chamber while the stem is moving from the origin position to the metered quantity discharge position, and is positioned in the storage region when the stem is positioned at the metered quantity discharge position.

### BRIEF DESCRIPTION OF THE DRAWINGS

Fig. 1 is a perspective view of a metering valve according to a first embodiment.
Fig. 2 is a front view of a metering valve.
Fig. 3 is a cross-sectional view of the metering valve taken along line A-A of Fig. 2.
Fig. 4 is a cross-sectional view of the metering valve taken along line B-B of Fig. 3.
Fig. 5 is an enlarged cross-sectional view of and around a metering chamber of the metering valve, which is taken along line A-A.
Fig. 6 is an enlarged cross-sectional view of and around the metering chamber of the metering valve, which is taken along line B-B.
Fig. 7 is a side cross-sectional view of a metering valve when a stem is arranged at a metered quantity discharge position.
Fig. 8 is an enlarged side cross-sectional view of a metering valve when a stem is arranged at a metered quantity discharge position.
Fig. 9 is a side cross-sectional view of a metering valve when a stem is arranged at a continuous discharge position.
Fig. 10 is an enlarged side cross-sectional view of a metering valve when a stem is arranged at a continuous discharge position.
Fig. 11(A) is a perspective view of a stem and Fig. 11(B) is a side cross-sectional view of the stem.

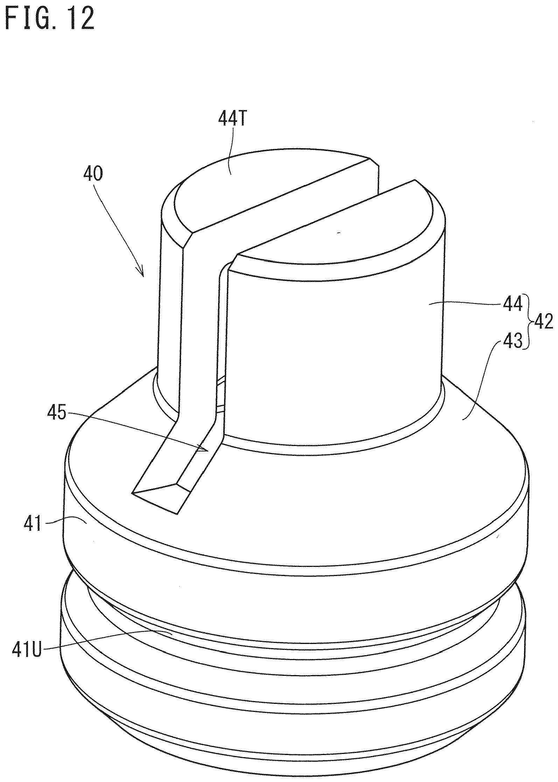

Fig. 12 is a perspective view of a cup-shaped fixing member.
Fig. 13 is a side cross-sectional view of a stem and a stopper arranged at a metered quantity discharge position in a metering valve according to another embodiment.
Fig. 14 is a side cross-sectional view of a stem arranged at a continuous discharge position in a metering valve according to another embodiment.
Fig. 15 is a side cross-sectional view of a stem arranged at a metered quantity discharge position in a metering valve according to another embodiment.
Fig. 16 is a side cross-sectional view of a stem arranged at a continuous discharge position after a mouthpiece is rotated by 90 degrees in a metering valve according to another embodiment.

### MODE FOR CARRYING OUT THE INVENTION

### [First embodiment]

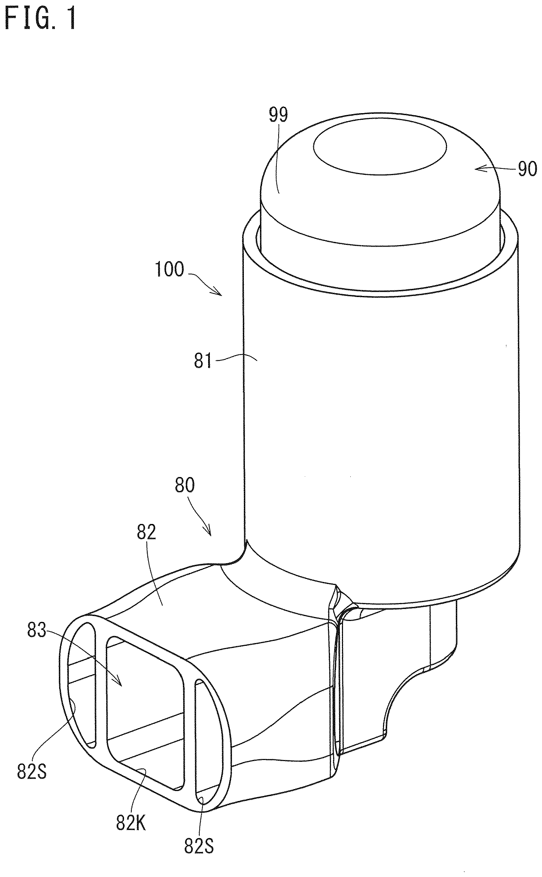

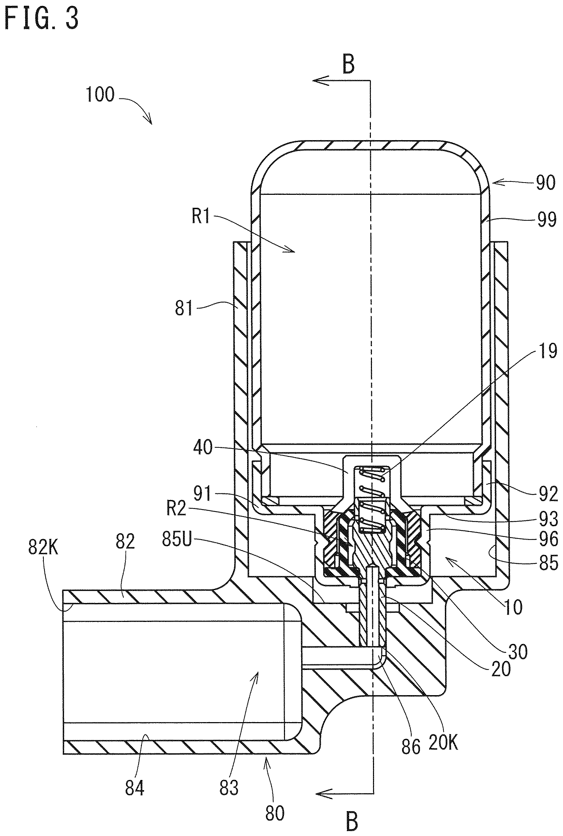

Figs. 1 and 3 illustrate a spray inhaler 100 including a metering valve 10 according to a first embodiment. The spray inhaler 100 is formed by fixing a high-pressure container 90 to a mouthpiece 80 from above. The spray inhaler 100 of this embodiment is used as, for example, an asthma treatment device for allowing an asthma patient to take a specific amount of asthma medication at a time. Specifically, when the high-pressure container 90 is pressed from above while the mouthpiece 80 is being held in a mouth, a drug such as asthma medication with which the high-pressure container 90 is filled is sprayed from a suction port 82K of the mouthpiece 80.

The high-pressure container 90 has a hollow cylindrical shape and has a storage region R1 isolated from an atmospheric pressure space. The storage region R1 is filled with a high-pressure fluid containing a drug and the like (hereinafter, referred to as a drug fluid as appropriate). The high-pressure fluid contains, for example, a chemical liquid and a compressed gas. The high-pressure container 90 is formed by fixing a cylindrical body 99 having one bottomed end and the other open end to a mounting cup 91 from above. Specifically, an outer peripheral surface of the other end (lower end) of the cylindrical body 99 is fitted into an inner surface of an outer peripheral wall 92 of the mounting cup 91 and is fixed thereto by, for example, welding. The cylindrical body 99 may also be fixed to the mounting cup 91 by, for example, clamping the outer peripheral wall of the mounting cup 91 inward.

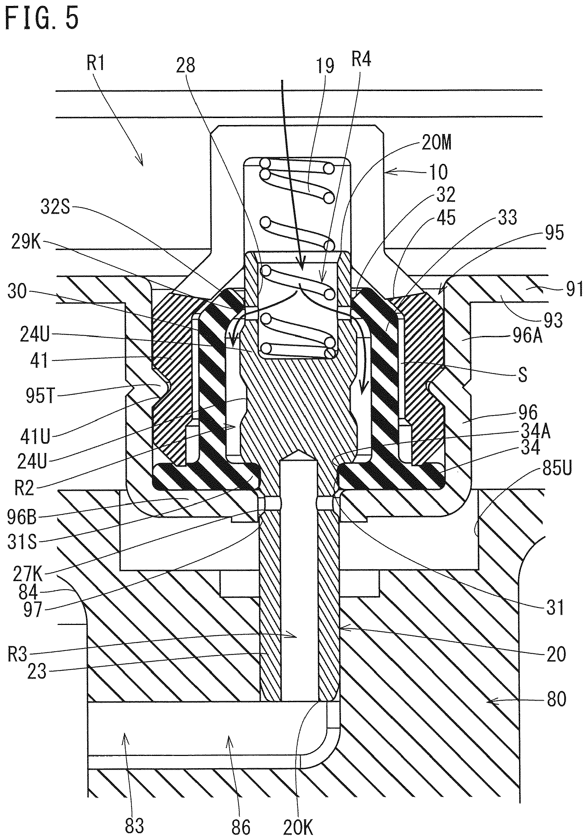

The mounting cup 91 has a bottom portion 93 that closes a lower end of the outer peripheral wall 92. At the center of the bottom portion 93, a downward protrusion 96 is provided to protrude downward so as to have a storage recess 95 on an inner surface of the mounting cup 91. The downward protrusion 96 has a first tubular wall 96A having a cylindrical shape and a first end wall 96B covering a distal end (lower end) of the first tubular wall 96A. A first fitting hole 97 is formed to penetrate the center of the first end wall 96B (see Fig. 5).

The mouthpiece 80 has an L shape as a whole and has, in a vertical side portion thereof, a container fitting portion 81 to which the high-pressure container 90 is fitted. The container fitting portion 81 has a cylindrical shape having an open upper end. The mouthpiece 80 has, in a lateral side portion thereof, a suction portion 82 to be held in a mouth of the asthma patient, for example. An L-shaped hole 83 bent in an L shape is formed inside the mouthpiece 80. One end of the L-shaped hole 83 forms the suction port 82K open to a distal end surface of the lateral side portion of the mouthpiece 80. In the metering valve 10, a high-pressure fluid L ejected from the high-pressure container 90 flows out from the suction port 82K through the L-shaped hole 83.

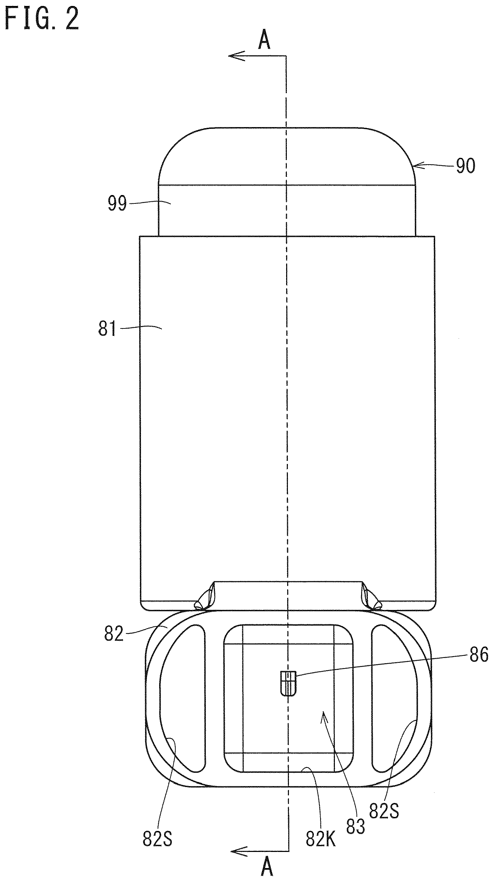

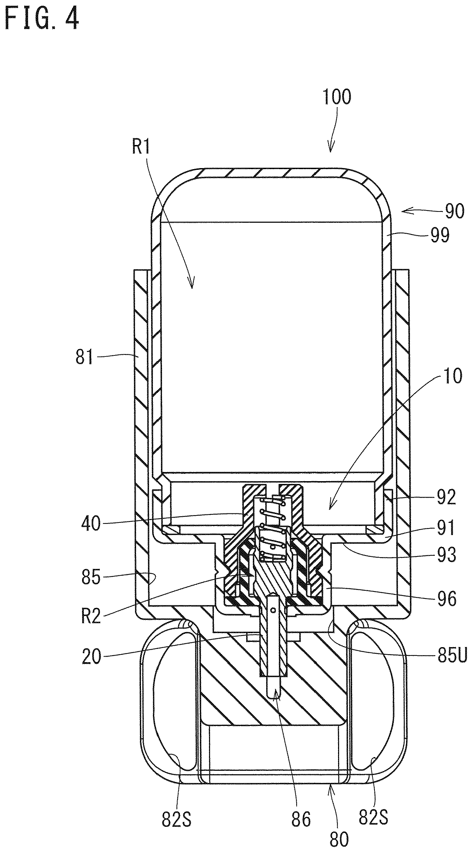

As illustrated in Figs. 2 and 4, side outside-air suction holes 82S are open on the left and right sides of the suction port 82K in a distal end surface of the suction portion 82 of the mouthpiece 80. The side outside-air suction holes 82S extend along a lateral side portion of the L-shaped hole 83 (for example, in parallel with the lateral side portion) to penetrate the mouthpiece 80. Because the side outside-air suction holes 82S are provided, for example, when the asthma patient sucks asthma medication from the suction port 82K while holding the suction portion 82 in the mouth, the asthma patient can also simultaneously suck outside air through the side outside-air suction holes 82S. Further, in this embodiment, the suction portion 82 of the mouthpiece 80 has a flat shape that laterally extends as seen from the distal end, and the side outside-air suction holes 82S are laterally aligned with the suction port 82K. This makes it possible to easily hold the suction port 82K in the mouth and also to easily suck outside air through the side outside-air suction holes 82S.

As illustrated in Fig. 3, the L-shaped hole 83 of the mouthpiece 80 has a suction-port-side square hole portion 84 expanding in a quadrangular cross section from an intermediate position on the lateral side portion of the L-shaped hole 83 toward the suction port 82K and also has a container-side enlarged diameter portion 85 having a circular cross section and expanding in diameter from an intermediate position in a vertical side portion of the L-shaped hole 83 toward a terminal end thereof. The container-side enlarged diameter portion 85 forms the inside of the container fitting portion 81. A small-diameter corner hole portion 86 having an L shape and having the smallest diameter is formed between the suction-port-side square hole portion 84 and the container-side enlarged diameter portion 85 in the L-shaped hole 83. A circular recess 85U is recessed in an enlarged diameter surface between the small-diameter corner hole portion 86 and the container-side enlarged diameter portion 85 (that is, a bottom surface of the container-side enlarged diameter portion 85 at a lower end of the container fitting portion 81).

In the metering valve 10 of this embodiment, the stem 20 protrudes through a lower surface of the mounting cup 91 of the high-pressure container 90 (specifically, a lower surface of the downward protrusion 96). The stem 20 has, at a protruding terminal end (lower end) thereof, a terminal end discharge port 20K for ejecting the drug fluid in the high-pressure container 90 into the outside air. The high-pressure container 90 is fitted into the container-side enlarged diameter portion 85 in the container fitting portion 81 of the mouthpiece 80. The terminal end of the stem 20 is fitted and fixed into a vertical side portion of the small-diameter corner hole portion 86 of the L-shaped hole 83 of the mouthpiece 80. The stem 20 is inserted into a round corner portion of the small-diameter corner hole portion 86, and a distal end of the stem 20 abuts on an inner surface of the corner portion. Thus, the stem 20 is positioned with respect to the mouthpiece 80. A distal end of the downward protrusion 96 of the mounting cup 91 is received by the circular recess 85U of the mouthpiece 80.

Here, as described above, when the high-pressure container 90 is pressed downward in the metering valve 10 of this embodiment, the drug fluid in the high-pressure container 90 is sprayed from the suction port 82K. Specifically, when the high-pressure container 90 is pressed, the stem 20 fixed in the small-diameter corner hole portion 86 of the mouthpiece 80 is pushed into the high-pressure container 90, and, when the stem 20 is pushed into the high-pressure container 90 in this manner, the drug fluid in the high-pressure container 90 is ejected.

Hereinafter, a structure of the metering valve 10 for ejecting the drug fluid will be described in detail. As illustrated in Fig. 5, a metering chamber R2 partitioned from the storage region R1 is provided inside the high-pressure container 90. The metering chamber R2 is stored in a storage recess 95 provided inside the downward protrusion 96 of the mounting cup 91. Specifically, a container-shaped seal member 30 is placed in the storage recess 95, and the metering chamber R2 is a region inside the container-shaped seal member 30.

The container-shaped seal member 30 has a cup portion 33 having a cup shape and a flange portion 34 covering an opening of the cup portion 33. The flange portion 34 has a larger diameter than the cup portion 33 and is placed on a bottom surface of the storage recess 95 (that is, to the first end wall 96B). A first seal hole 31 extending over the metering chamber R2 and the atmospheric pressure space (outside air) penetrates the center of the flange portion 34. In this embodiment, the first seal hole 31 is formed by connecting a center hole 34A of the flange portion 34 and the first fitting hole 97 of the mounting cup 91 described above. Further, a second seal hole 32 extending over the metering chamber R2 and the storage region R1 is formed to penetrate the center of a bottom portion of the cup portion 33. The first seal hole 31 and the second seal hole 32 are coaxially arranged. In the example of this embodiment, the container-shaped seal member 30 is an integrally molded product and is made of, for example, an elastomer.

The container-shaped seal member 30 is fixed by a cup-shaped fixing member 40. The cup-shaped fixing member 40 is arranged to cover the container-shaped seal member 30 and is fixed while being fitted to an inner peripheral surface of the storage recess 95. The cup-shaped fixing member 40 sandwiches the flange portion 34 of the container-shaped seal member 30 between an end of an opening of the cup-shaped fixing member 40 and the bottom surface of the storage recess 95 (that is, the first end wall 96B), thereby fixing the container-shaped seal member 30 to the mounting cup 91. An outer peripheral surface of the cup-shaped fixing member 40 and the inner peripheral surface of the storage recess 95 (that is, an inner peripheral surface of the first tubular wall 96A) have protrusion-recess engagement portions whose protrusion and recess engage with each other. Specifically, the protrusion-recess engagement portions are formed by an annular groove 41U formed on the outer peripheral surface of the cup-shaped fixing member 40 and an annular protrusion 95T formed on the inner peripheral surface of the storage recess 95 (that is, of the first tubular wall 96A). For example, the annular protrusion 95T of the first tubular wall 96A can be formed by crimpling the first tubular wall 96A from the outside. The protrusion-recess engagement portions described above may also be formed by a protrusion (e.g., an annular protrusion) formed on the outer peripheral surface of the cup-shaped fixing member 40 and a recess (e.g., an annular groove) formed on the inner peripheral surface of the storage recess 95 (the first tubular wall 96A).

The cup-shaped fixing member 40 has a fitting tubular wall 41 having a cylindrical shape and having the annular groove 41U and a head portion 42 covering an end of the fitting tubular wall 41. The head portion 42 has a tapered wall 43 that is connected to the fitting tubular wall 41 and is reduced in diameter as the tapered wall 43 separates away from the fitting tubular wall 41 and a cylindrical portion 44 connected to the tapered wall 43 and having one bottomed end. A slit 45 is formed to penetrate the head portion 42. Specifically, when viewed in an axial direction of the cup-shaped fixing member 40, the slit 45 radially extends to cross the cylindrical portion 44, and both ends of the slit 45 extend toward radial intermediate positions of the tapered wall 43. In this embodiment, for example, the slit 45 is arranged to have a central axis of the cylindrical portion 44 and divides the cylindrical portion 44 into two equal parts. The cup-shaped fixing member 40 may be made of, for example, resin. In this case, the slit 45 is formed to have a shape extending straight in the axial direction of the cup-shaped fixing member 40. This makes it easier to remove a molding die and to form the cup-shaped fixing member 40 by using resin.

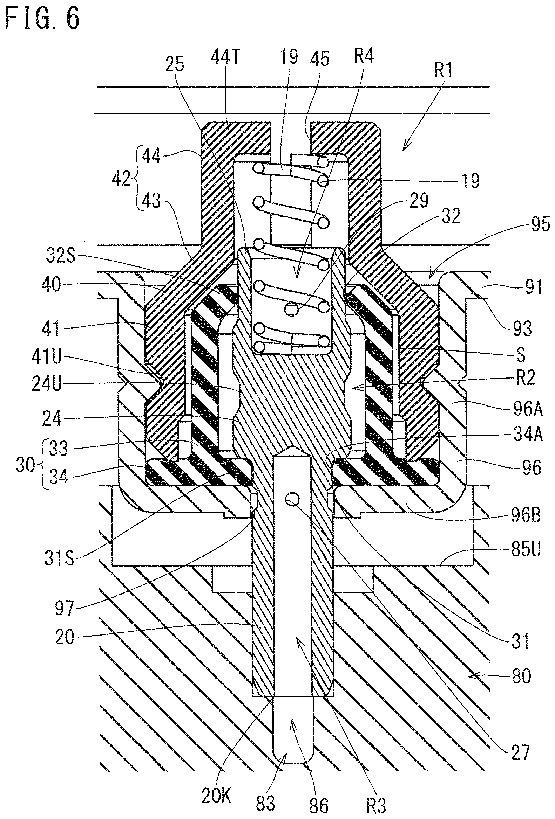

As illustrated in Figs. 5 and 6, a gap S is formed between an inner peripheral surface of the cup-shaped fixing member 40 and an outer peripheral surface of the container-shaped seal member 30 (specifically, an outer peripheral surface of the cup portion 33). The gap S has a substantially tubular shape and allows the drug fluid in the storage region R1 to enter through the slit 45 of the cup-shaped fixing member 40. The drug fluid applies pressure to the container-shaped seal member 30 (that is, the metering chamber R2) from the outside.

The stem 20 described above is inserted into the first seal hole 31 and the second seal hole 32 and penetrates the metering chamber R2 (that is, extends in a direction in which the bottom portion 93 of the mounting cup 91 and the cup portion 33 face each other). The stem 20 is supported to be linearly movable with respect to the metering chamber R2. Specifically, the first seal hole 31 supports a first intermediate portion 21 of the stem 20 so that the first intermediate portion 21 is linearly movable, and an end of an opening of the center hole 34A of the flange portion 34 in the first seal hole 31 forms a first seal portion 31S that closes a gap between the end of the opening and the stem 20. The second seal hole 32 supports a second intermediate portion 22 of the stem 20 so that the second intermediate portion 22 is linearly movable, and an end of an opening of the second seal hole 32 forms a second seal portion 32S that closes a gap between the end of the opening and the stem 20. In the present disclosure, the intermediate portion of the stem 20 means an intermediate portion in an axial direction of the stem 20.

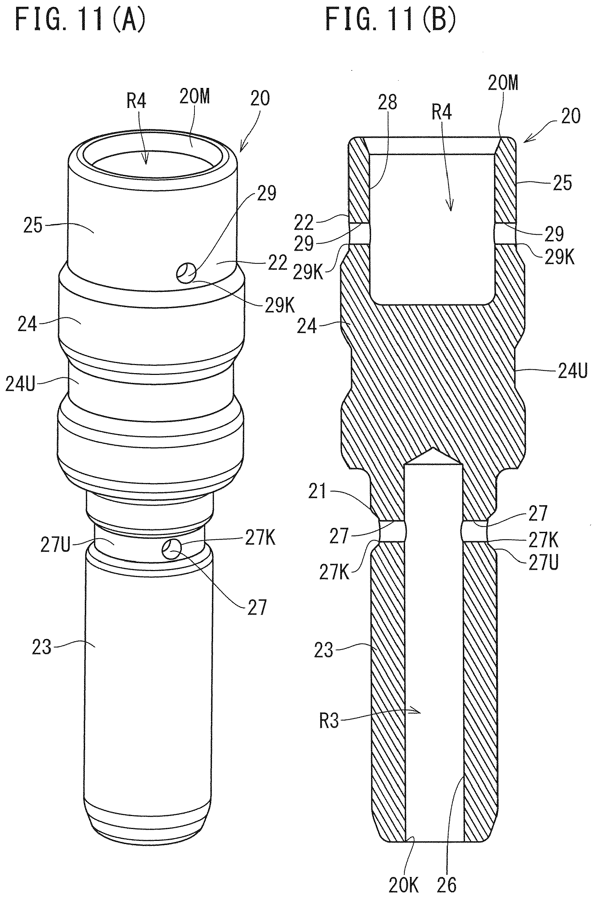

As illustrated in Figs. 11(A) and 11(B), in the example of this embodiment, the stem 20 has a cylindrical shape and is formed by axially connecting a small diameter portion 23, a large diameter portion 24, and a medium diameter portion 25 from the terminal end that is always arranged in the atmospheric pressure space toward its starting end that is always arranged in the storage region R1. The small diameter portion 23 extends from the terminal end of the stem 20 toward the center in the axial direction thereof. The large diameter portion 24 has a larger diameter than the small diameter portion 23 and has, at an axial intermediate portion thereof, an annular groove 24U extending in a peripheral direction of the large diameter portion 24. The medium diameter portion 25 has a larger diameter than the small diameter portion 23, has a smaller diameter than the largest diameter of the large diameter portion 24, and has a slightly larger diameter than an outer shape of a bottom of the annular groove 24U.

A portion of the small diameter portion 23 close to the large diameter portion 24 (close to the starting end) forms the above-described first intermediate portion 21 that is supported by the first seal hole 31 so as to be linearly movable. In the stem 20, a linear hole portion 26 is formed to axially extend from the terminal end of the stem 20 (that is, a terminal end of the small diameter portion 23) toward an intermediate position closer to the starting end than the first intermediate portion 21. In the first intermediate portion 21 of the stem 20, radially extending side holes 27 are formed. The side holes 27 communicate with the linear hole portion 26 and have a peripheral surface inlet port 27K that is open to an outer peripheral surface of the first intermediate portion 21. The linear hole portion 26 and the side holes 27 form a terminal-end-side stem internal flow path R3 extending from the terminal end of the stem 20 toward the first intermediate portion 21. In the example of this embodiment, the side holes 27 are arranged at two positions separated by 180 degrees in the peripheral direction of the stem 20. Further, an annular groove 27U is formed in a portion of the stem 20 where the side holes 27 are provided, and the peripheral surface inlet ports 27K are arranged on a bottom of the annular groove 27U.

A portion of the medium diameter portion 25 close to the large diameter portion 24 (close to the terminal end) forms the above-described second intermediate portion 22 that is supported by the second seal hole 32 so as to be linearly movable. In the stem 20, a linear hole portion 28 is formed to axially extend from the starting end of the stem 20 (that is, a starting end of the medium diameter portion 25) toward an intermediate position closer to the terminal end than the second intermediate portion 22. In the second intermediate portion 22 of the stem 20, radially extending side holes 29 are formed. The side holes 29 communicate with the linear hole portion 28 and have a peripheral surface discharge port 29K that is open to an outer peripheral surface of the second intermediate portion 22. The linear hole portion 28 and the side holes 29 form a starting-end-side stem internal flow path R4 extending from the starting end of the stem 20 toward the second intermediate portion 22. In the example of this embodiment, the side holes 29 are arranged at two positions separated by 180 degrees in the peripheral direction of the stem 20, but the number and arrangement of the side holes 29 are not limited thereto.

When the stem 20 is attached to penetrate the first seal hole 31 and the second seal hole 32 as illustrated in Fig. 6, the starting end of the stem 20 is received inside the cylindrical portion 44 of the cup-shaped fixing member 40. The linear hole portion 28 of the stem 20 receives an elastic member 19. The elastic member 19 is compressed between a ceiling portion 44T of the cylindrical portion 44 axially facing the stem 20 and a bottom surface of the linear hole portion 28 of the stem 20 and biases the stem 20 toward an origin position (see Fig. 3). Thus, in this embodiment, the elastic member 19 is arranged outside the metering chamber R2. In the example of this embodiment, the elastic member 19 includes a compression coil spring extending in the axial direction of the stem 20, but the elastic member 19 is not limited to the compression coil spring.

As illustrated in Fig. 5 (Fig. 3), the stem 20 is biased to the outside of the high-pressure container 90 (downward) and is positioned at the origin position. In this embodiment, the stem 20 is biased by the elastic member 19 and is also biased by the high-pressure fluid in the storage region R1 through the slit 45 of the cup-shaped fixing member 40. Therefore, even in a case where the elastic member 19 is not provided, the stem 20 is biased to the outside of the high-pressure container 90 by the high-pressure fluid. The origin position is a position where the stem 20 protrudes at the lowest position. At the origin position, an enlarged diameter surface at a lower end of the large diameter portion 24 of the stem 20 is retained on the metering chamber R2 side by an edge of the opening of the first seal hole 31 of the container-shaped seal member 30. Therefore, the stem 20 is normally positioned at the origin position.

When the stem 20 is at the origin position, the metering chamber R2 and the atmospheric pressure space are blocked, and the metering chamber R2 and the storage region R1 communicate with each other. Therefore, at this time, the metering chamber R2 is filled with the drug fluid from the storage region R1. Specifically, the terminal-end-side stem internal flow path R3 of the stem 20 is arranged in the atmospheric pressure space, and there is no space between the stem 20 and the inner surface of the first seal hole 31 of the container-shaped seal member 30. Meanwhile, the starting-end-side stem internal flow path R4 of the stem 20 causes the metering chamber R2 and the storage region R1 to communicate with each other. That is, the peripheral surface discharge ports 29K of the starting-end-side stem internal flow path R4 are arranged in the metering chamber R2, and a starting end opening (a starting end inlet port 20M) of the starting-end-side stem internal flow path R4 (of the linear hole portion 28) is arranged in the storage region R1. At this time, there is no space between a portion of the medium diameter portion 25 of the stem 20, the portion being closer to the starting end of the stem 20 than the peripheral surface discharge ports 29K, and an inner surface of the second seal hole 32 of the container-shaped seal member 30. When the stem 20 is arranged at the origin position, the lower surface of the downward protrusion 96 of the high-pressure container 90 and a bottom surface of the circular recess 85U of the mouthpiece 80 are separate from each other.

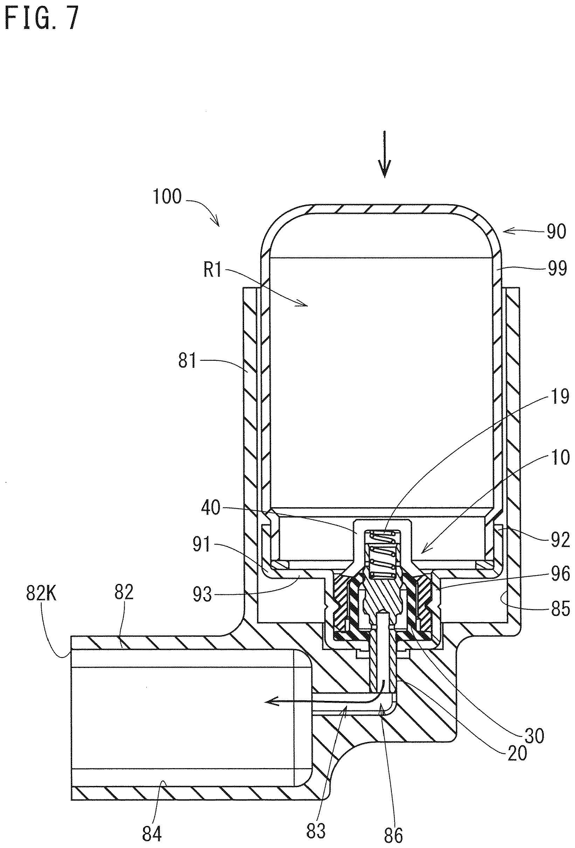

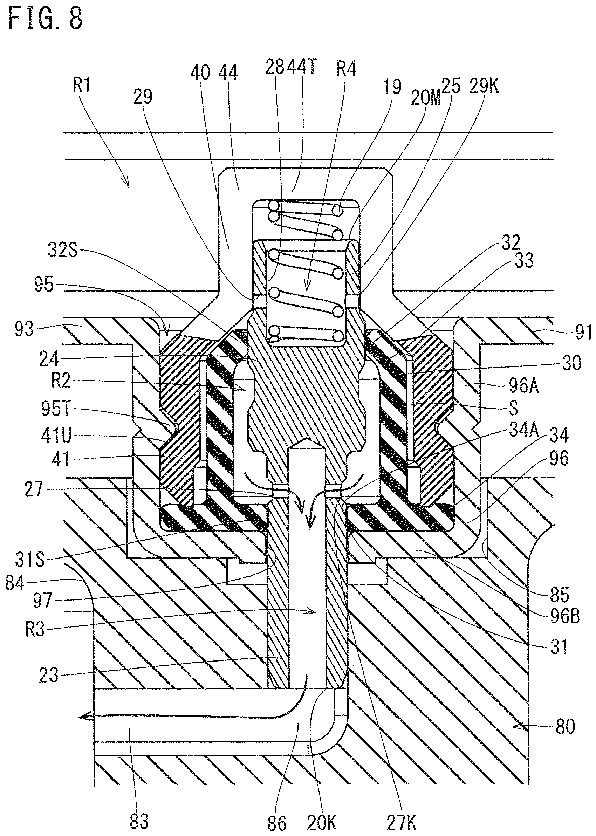

Here, when the high-pressure container 90 is pressed from above as illustrated in Fig. 8 (Fig. 7), the high-pressure container 90 approaches the mouthpiece 80, and the stem 20 is pushed into the high-pressure container 90. Then, the lower surface of the downward protrusion 96 of the high-pressure container 90 abuts on the bottom surface of the circular recess 85 of the mouthpiece 80, thereby positioning the stem 20. The position of the stem with respect to the metering chamber R2 in this state is a metered quantity discharge position.

At the metered quantity discharge position, the metering chamber R2 and the storage region R1 are blocked, the metering chamber R2 and the atmospheric pressure space communicate with each other, and the drug fluid in the metering chamber R2 is ejected by a volume of the metering chamber R2 into the L-shaped hole 83 of the mouthpiece 80 through the terminal-end-side stem internal flow path R3. Therefore, it is possible to suck a specific amount of drug from the suction port 82K through the L-shaped hole 83. In the metering valve 10 of this embodiment, the container-shaped seal member 30 (that is, the metering chamber R2) is pushed from the outside by the drug fluid that has entered the gap S through the slit 45 of the cup-shaped fixing member 40, and an external pressure of the cup-shaped fixing member 40 is higher than an internal pressure thereof. Therefore, when the stem 20 reaches the metered quantity discharge position, the drug fluid can be stably ejected from the metering chamber R2.

Specifically, at the metered quantity discharge position, the starting-end-side stem internal flow path R4 of the stem 20 is arranged in the storage region R1, and there is no space between the stem 20 and the inner surface of the second seal hole 32 of the container-shaped seal member 30. Meanwhile, the terminal-end-side stem internal flow path R3 of the stem 20 causes the metering chamber R2 and the atmospheric pressure space to communicate with each other. That is, the peripheral surface inlet ports 27K of the terminal-end-side stem internal flow path R3 are arranged in the metering chamber R2, and the terminal end discharge port 20K at a terminal end of the terminal-end-side stem internal flow path R3 (of the linear hole portion 26) is arranged in the atmospheric pressure space. At this time, there is no space between a portion of the small diameter portion 23 of the stem 20, the portion being closer to the terminal end of the stem 20 than the peripheral surface inlet ports 27K, and the inner surface of the first seal hole 31 of the container-shaped seal member 30. At this time, the starting end of the stem 20 is fitted into the cylindrical portion 44 of the cup-shaped fixing member 40. While the movement of the stem 20 is moving from the origin position to the metered quantity discharge position, the peripheral surface discharge ports 29K move from the metering chamber R2 to the storage region R1 before the peripheral surface inlet ports 27K are positioned in the metering chamber R2.

Thereafter, when the press of the high-pressure container 90 is stopped, the stem 20 is arranged at the origin position again by the pressure of the drug fluid and biasing force of the elastic member 19, and the metering chamber R2 is filled with the drug fluid. In the metering valve 10 of this embodiment, it is possible to repeatedly spray a metered quantity of drug by repeating an operation of pressing the high-pressure container 90 from above. When the metered quantity of drug fluid is sprayed from the metering chamber R2, the container-shaped seal member 30 may be deformed inward by the pressure of the drug fluid in the storage region R1 (in the gap S). However, even in this case, when the stem 20 returns to the origin position, the shape of the container-shaped seal member 30 is restored by the pressure of the drug fluid that has flowed into the metering chamber R2 and elasticity of the container-shaped seal member 30. Thus, the metering chamber R2 can be maintained at a constant volume.

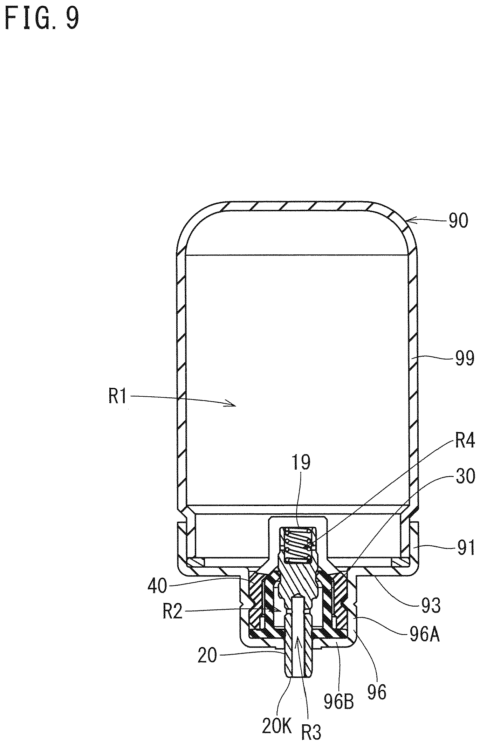

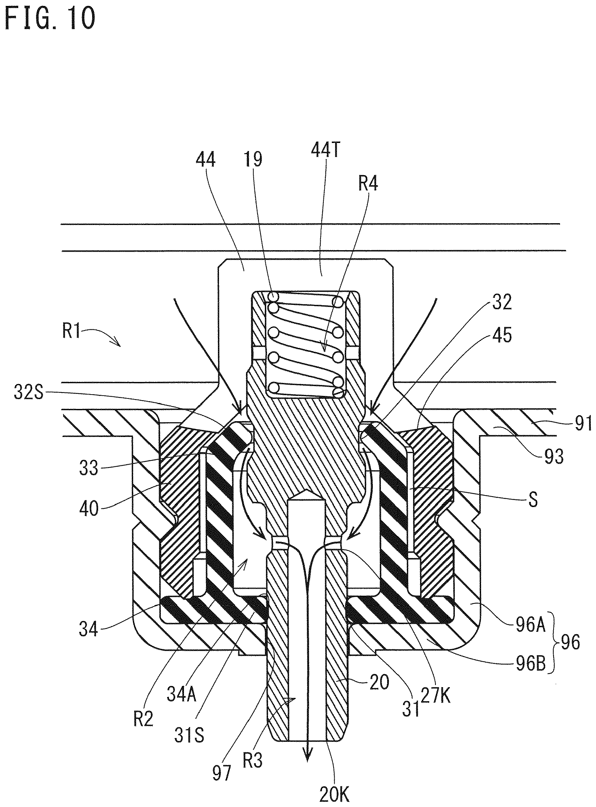

Here, in the metering valve 10 of this embodiment, the high-pressure container 90 can be removed from the mouthpiece 80 (see Fig. 9). Specifically, the high-pressure container 90 can be removed by pulling out the stem 20 from the L-shaped hole 83 of the mouthpiece 80. At this time, as illustrated by a change from Fig. 8 to Fig. 10, the stem 20 can be further pushed into the high-pressure container 90 from the metered quantity discharge position. Specifically, as illustrated in Figs. 9 and 10, the stem 20 can be pushed until a starting end surface of the stem 20 abuts on the ceiling portion 44T of the cylindrical portion 44 of the cup-shaped fixing member 40. In this embodiment, the position of the stem 20 with respect to the metering chamber R2 in this state is referred to as a continuous discharge position. As illustrated in Fig. 10, at the continuous discharge position, the storage region R1 and the atmospheric pressure space communicate with each other, and the drug fluid in the high-pressure container 90 can be continuously ejected into the atmospheric pressure space.

Specifically, at the continuous discharge position, the peripheral surface inlet ports 27K of the stem 20 are arranged in the metering chamber R2 and are arranged in the atmospheric pressure space through the terminal end discharge port 20K at the terminal end of the stem 20. This makes it possible to cause the metering chamber R2 and the atmospheric pressure space to communicate with each other through the terminal-end-side stem internal flow path R3. Further, at the continuous discharge position, the annular groove 24U of the large diameter portion 24 of the stem 20, which is arranged in the metering chamber R2 at the metered quantity discharge position, is arranged in the second seal hole 32 of the container-shaped seal member 30. Here, an outer diameter of the bottom of the annular groove 24U of the stem 20 is smaller than an outer diameter of the medium diameter portion 25 of the stem 20. Thus, there is a gap between the inner surface of the second seal hole 32 and an inner surface of the annular groove 24U, and this gap causes the storage region R1 and the metering chamber R2 to communicate with each other. In this embodiment, the annular groove 24U forms a seal path portion to cause the storage region R1 and the metering chamber R2 to communicate with each other over the second seal portion 32S.

When the stem 20 is arranged at the continuous discharge position, the storage region R1 and the atmospheric pressure space communicate with each other. Therefore, in a case where all the drug fluid in the storage region R1 flows out, it is also possible to fill the storage region R1 with the drug fluid from the terminal end discharge port 20K of the stem 20.

Hereinabove, the configuration of the metering valve 10 of this embodiment has been described. In this embodiment, the bottom portion 93 of the mounting cup 91 and the cup portion 33 of the container-shaped seal member 30 correspond to a "high-pressure partition wall" and an "internal partition wall", respectively, recited in the claims. The cup-shaped fixing member 40 and the flange portion 34 of the container-shaped seal member 30 correspond to a "stem receiving cup" and a "second seal member", respectively, recited in the claims. The slit 45 corresponds to a "communication hole" and a "fixing member communication hole" recited in the claims. The position of the high-pressure container 90 when the stem 20 is at the origin position and the position of the high-pressure container 90 when the stem is at the metered quantity discharge position correspond to a "first position" and a "second position", respectively, recited in the claims.

Next, effects of the metering valve 10 of this embodiment will be described. In the metering valve 10 of this embodiment, the first intermediate portion 21 of the stem 20 is supported to be linearly movable by the first seal hole 31 extending over the atmospheric pressure space and the metering chamber R2. The second intermediate portion 22 of the stem 20 is supported to be linearly movable by the second seal hole 32 extending over the storage region R1 and the metering chamber R2 of the high-pressure container 90. Here, the starting-end-side stem internal flow path R4 provided in the stem 20 causes the storage region R1 and the metering chamber R2 to communicate with each other when the stem 20 is arranged at the origin position. Therefore, when the stem 20 is arranged at the origin position, the drug fluid flows into the metering chamber R2 from the storage region R1 through the starting-end-side stem internal flow path R4 of the stem 20 inserted into the second seal hole 32. Thus, the metering chamber R2 is filled the drug fluid. This eliminates the necessity of providing a gap through which the drug fluid passes between the inner peripheral surface of the second seal hole 32 and the outer peripheral surface of the stem 20 when the metering chamber R2 is filled with the drug fluid. Therefore, the second seal hole 32 can improve stability of guiding and supporting the stem 20 at the time of spraying the metered quantity of drug fluid.

In the metering valve 10, both the first seal portion 31S and the second seal portion 32S are provided in the container-shaped seal member 30, and thus the number of parts can be reduced as compared with a case where the first seal portion 31S and the second seal portion 32S are provided in separate members. This makes it possible to improve assembling efficiency of the seal member.

In the metering valve 10, the container-shaped seal member 30 is received by the storage recess 95 of the bottom portion 93 of the mounting cup 91 and is fixed by being covered by the cup-shaped fixing member 40. Then, the container-shaped seal member 30 has the flange portion 34 to be fitted to the bottom surface of the storage recess 95, and the flange portion 34 is fixed by being sandwiched between the bottom surface of the storage recess 95 and an open end of the cup-shaped fixing member 40. This makes it possible to stabilize the fixation of the container-shaped seal member 30. Because the cup-shaped fixing member 40 is fitted into the storage recess 95 inside the downward protrusion 96 of the mounting cup 91, the cup-shaped fixing member 40 and the container-shaped seal member 30 can be easily fixed. The inner peripheral surface of the storage recess 95 and the outer peripheral surface of the cup-shaped fixing member 40 have the protrusion-recess engagement portions (the annular protrusion 95T and the annular groove 41U) whose protrusion and recess engage with each other. In this way, the cup-shaped fixing member 40 and the container-shaped seal member 30 can be easily fixed to the mounting cup 91.

In the metering valve 10, the elastic member 19 compressed between the ceiling portion 44T of the cup-shaped lake bottom member 40 and the stem 20 can easily bias the stem 20 to the origin position. The elastic member 19 is arranged in the storage region R1 with respect to the internal partition wall (the cup portion 34 of the container-shaped seal member 30) that partitions the storage region R1 and the metering chamber R2, and thus the elastic member 19 is arranged outside the metering chamber R2. Therefore, as compared with a case where the elastic member 19 is arranged in the metering chamber R2, the inside of the metering chamber R2 is less likely to have a complicated shape, and an ejection amount of the high-pressure fluid is easily stabilized. For example, in a case where a compression coil spring is used as the elastic member 19 and when the compression coil spring is arranged in the metering chamber R2, the inside of the metering chamber R2 is likely to have a complicated shape. This may make it difficult to stabilize the ejection amount of the high-pressure fluid. Meanwhile, in the metering valve 10 of this embodiment, the compression coil spring is arranged outside the metering chamber R2. This makes it possible to easily stabilize the ejection amount of the high-pressure fluid. The compression coil spring may be arranged between the stem 20 and the cup-shaped fixing member 40 that receives the stem 20 from the starting end thereof.

When the stem 29 is arranged at the continuous discharge position farther from the origin position than the metered quantity discharge position, the metering valve 10 can cause the storage region R1 in the high-pressure container 90 to communicate with the atmospheric pressure space through the metering chamber R2, a flow path in the stem 20, and the seal path portion (the annular groove 24U). Therefore, when the stem 20 is arranged at the continuous arrangement position, it is possible to continuously eject the high-pressure fluid in the high-pressure container 90. On the contrary, for example, when the high-pressure container 90 is empty, it is also possible to fill the storage region R1 in the high-pressure container 90 with the fluid from the outside through the stem 20 and the metering chamber R2. In this embodiment, the seal path portion that causes the storage region R1 and the metering chamber R2 to communicate with each other over the second seal portion 32S is configured as a recess (the annular groove 24U) formed in the outer peripheral surface of the stem 20, and thus the seal path portion can be easily formed.

By removing the mouthpiece 80 in the metering valve 10, a state of ejecting a specific amount of high-pressure fluid at a time can be switched to a state of continuously ejecting the high-pressure fluid.

### [Other embodiments]

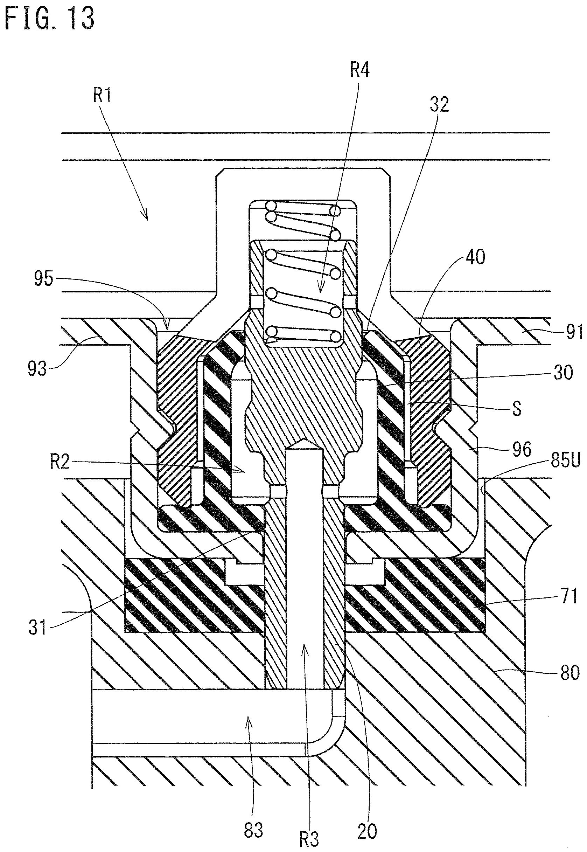

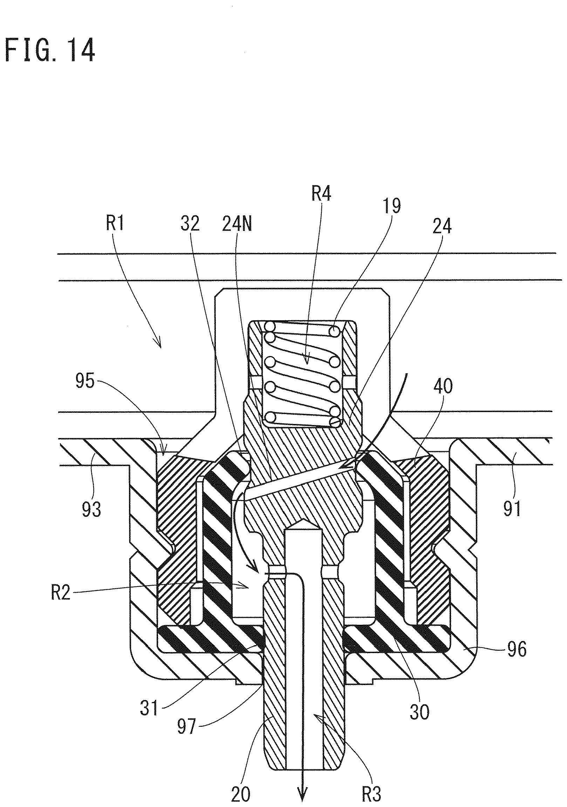

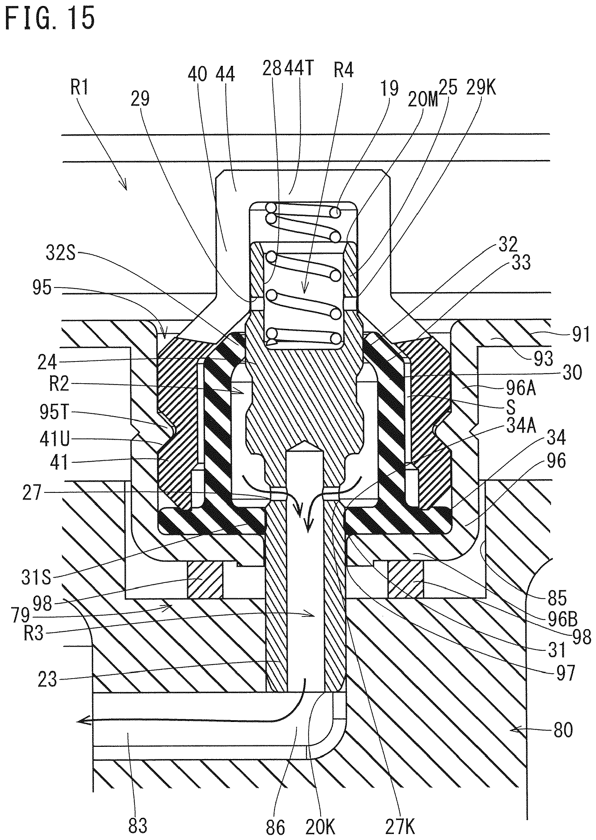

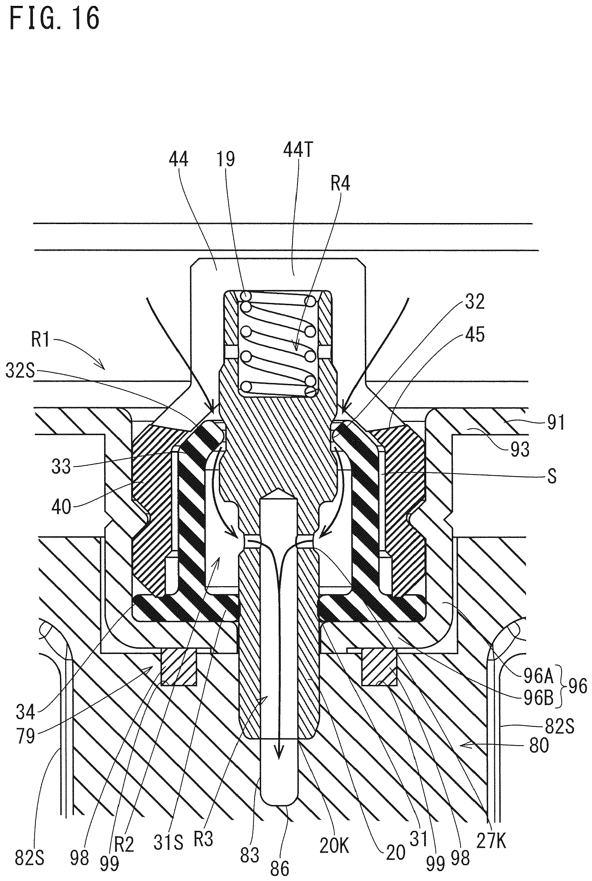

(1) In the above embodiment, the metering valve 10 is provided in a medical instrument, but the present disclosure is not limited thereto and may be provided in sprays for various uses, for example. The metering valve 10 may also be provided for discharging a disinfectant solution. In the metering valve 10 of the above embodiment, the high-pressure container 90 is provided on the upper side, but the high-pressure container 90 may be provided on the lower side, the right side, the left side, the front side, or the back side.
(2) In the above embodiment, an opening at the terminal end of the terminal-end-side stem internal flow path R3 in the stem 20 (the terminal end discharge port 20K of the stem 20) is arranged in a terminal end surface of the stem 20, but may be arranged in an outer peripheral surface of the terminal end portion of the stem 20. Further, an opening at the starting end of the starting-end-side stem internal flow path R4 in the stem 20 (the starting end inlet port 20M) is arranged in a starting end surface of the stem 20, but may be arranged in an outer peripheral surface of the starting end portion of the stem 20.
(3) As illustrated in Fig. 13, a stopper 71 made from an elastic member may be arranged on a bottom surface portion of the storage recess 95 of the mounting cup 91. In this configuration, when the stem 20 is pushed into the metered quantity discharge position, the lower surface of the downward protrusion 96 of the mounting cup 91 abuts on the bottom surface of the circular recess 85U of the mouthpiece 80, thereby positioning the stem 20 at the metered quantity discharge position. When the stem 20 is further strongly pushed into the high-pressure container 90, the stopper 71 is compressed and elastically deformed by the downward protrusion 96, and thus the stem 20 can be pushed until the stem 20 abuts on the ceiling portion 44T of the cup-shaped fixing member 40. With this configuration, it is possible to selectively use the metering valve 10 in a metered quantity ejection mode in which a specific amount of high-pressure fluid in the high-pressure container 90 is ejected at a time or a continuous ejection mode in which the high-pressure fluid is continuously ejected.
(4) In the above embodiment, the seal path portion that causes the storage region R1 and the metering chamber R2 to communicate with each other over the second seal portion 32S at the continuous discharge position is the annular groove 24U of the stem 20, but may be a recess such as a groove extending in the axial direction of the stem 20. As illustrated in Fig. 14, the seal path portion may be an inclined hole 24N formed to penetrate the large diameter portion 24 of the stem 20.
(5) A mode switching mechanism 79 may be provided to switch between a first state in which the stem 20 is allowed to be pressed to the metered quantity discharge position and is restricted not to be further pressed and a second state in which the stem 20 is allowed to be pressed to the continuous discharge position. For example, as illustrated in Figs. 15 and 16, the above mode switching mechanism 79 including protrusions 98 and recesses 99 can be achieved by causing the protrusions 98 to protrude from the lower surface of the downward protrusion 96 of the high-pressure container 90, providing the recesses 99 in the bottom surface of the circular recess 85U of the mouthpiece 80 (see Fig. 16), and changing whether or not the protrusions 98 are received by the recesses depending on whether or not the mouthpiece 80 is rotated by 90 degrees with respect to the high-pressure container 90. Specifically, the protrusions 98 are arranged at, for example, two positions separated by 180 degrees around the axis of the stem 20. For example, in the arrangement of the mouthpiece 80 illustrated in Figs. 1 and 2, the protrusions 98 are not received by the recesses 99 and serve as spacers between the high-pressure container 90 and the mouthpiece 80 as illustrated in Fig. 15, and the stem 20 is allowed to be arranged from the origin position only to the metered quantity discharge position (first state). When the mouthpiece 80 is rotated by 90 degrees around the axis of the high-pressure container 90 from the arrangement of Figs. 1 and 2 (see Fig. 16), the protrusions 98 are received by the recesses 99, and the stem 20 is allowed to be arranged from the origin position to the continuous discharge position (second state). The stem 20 is not positioned at the metered quantity discharge position in the second state, and thus it is possible to push the stem 20 into the continuous discharge position at once.
(6) In the above embodiment, the downward protrusion 96 is provided in the bottom portion 93 of the mounting cup 91, but the bottom portion 93 may be flat. In this case, for example, a projection may be provided to project outward from the end of the opening of the cup-shaped fixing member 40, and the projection may be fixed to the bottom portion 93 of the mounting cup 91.
(7) In the above embodiment, a plurality of through-holes may be formed in the cup-shaped fixing member 40, instead of the slit. As the through-holes, it is preferable to provide a through-hole that causes the starting-end-side stem internal region R4 of the stem 20 and the storage region R1 to communicate with each other to bias the stem 20 toward the origin position with the high-pressure fluid and also provide a through-hole that causes the storage region R1 and the gap S outside/inside the cup-shaped fixing member 40 to communicate with each other. Such through-holes may be, for example, orifices formed by cutting. In a case where the slit 45 is formed in the cup-shaped fixing member 40 as in the metering valve 10 of the first embodiment, there is no need to provide such the plurality of through-holes. Thus, the cup-shaped fixing member 40 can be easily formed.
(8) In the above embodiment, the side outside-air suction holes 82S are provided in the mouthpiece 80, but the side outside-air suction holes 82S may not be provided.
(9) The mouthpiece 80 having the side outside-air suction holes 82S in the suction portion 82 may be attached to, for example, a high-pressure container having a conventional metering valve and may be used for, for example, a medical spray inhaler. Because the side outside-air suction holes 82S are provided, when a drug or the like is sucked from the suction portion 82 while the suction portion 82 is being held in the mouth, outside air can also be simultaneously sucked through the side outside-air suction holes 82S, which is preferable.

### DESCRIPTION OF THE REFERENCE NUMERAL

- 10: metering valve
- 19: elastic member
- 20: stem
- 20K: terminal end discharge port
- 21: first intermediate portion
- 22: second intermediate portion
- 23: small diameter portion
- 24: large diameter portion
- 24U: annular groove
- 25: medium diameter portion
- 27: side hole
- 27K: peripheral surface inlet port
- 27U: annular groove
- 29: side hole
- 29K: peripheral surface discharge port
- 30: container-shaped seal member
- 31: first seal hole
- 31S: first seal portion
- 32: second seal hole
- 32S: second seal portion
- 33: cup portion
- 34: flange portion
- 40: cup-shaped fixing member
- 41: fitting tubular wall
- 41U: annular groove
- 42: head portion
- 44: cylindrical portion
- 44T: ceiling portion
- 45: slit
- 80: mouthpiece
- 81: high-pressure container fitting portion
- 82: suction portion
- 82K: suction port
- 82S: side outside-air suction hole
- 83: L-shaped hole
- 85: circular recess
- 85U: circular recess
- 90: high-pressure container
- 91: mounting cup
- 92: outer peripheral wall
- 93: bottom portion
- 95: storage recess
- 95T: annular protrusion
- 96: downward protrusion
- 96A: first tubular wall
- 96B: first end wall
- 97: first fitting hole
- 100: spray inhaler
- R1: storage region
- R2: metering chamber
- R3: terminal-end-side stem internal flow path
- R4: starting-end-side stem internal flow path
- S: gap

## Claims

1. A metering valve comprising:
a high-pressure partition wall that forms part of a high-pressure container that isolates a storage region to be filled with a high-pressure fluid from an atmospheric pressure space;
an internal partition wall that is arranged inside the high-pressure partition wall and forms a metering chamber partitioned from the storage region between the internal partition wall and the high-pressure partition wall;
a stem that extends in a direction in which the high-pressure partition wall and the internal partition wall face each other, is linearly movably supported, and is biased toward the high-pressure partition wall to be positioned at an origin position;
a first seal hole that penetrates the high-pressure partition wall so as to extend over the atmospheric pressure space and the metering chamber and has a first seal portion linearly movably supporting a first intermediate portion of the stem and closing a gap between the first seal hole and the stem;
a second seal hole that penetrates the internal partition wall so as to extend over the metering chamber and the storage region and has a second seal portion linearly movably supporting a second intermediate portion of the stem and closing a gap between the second seal hole and the stem;
a terminal-end-side stem internal flow path that is formed inside the stem and extends from a terminal end of the stem to the first intermediate portion, the terminal end being always positioned in the atmospheric pressure space;
a starting-end-side stem internal flow path that is formed inside the stem and extends from a starting end of the stem to the second intermediate portion, the starting end being always positioned in the storage region;
a terminal end discharge port that is provided in the terminal-end-side stem internal flow path and is open to the terminal end of the stem;
a peripheral surface inlet port that is provided in the terminal-end-side stem internal flow path, is open to an outer peripheral surface of the first intermediate portion of the stem, is positioned to be closer to the atmospheric pressure space than to the metering chamber when the stem is arranged at the origin position, and is positioned in the metering chamber when the stem is moved to a metered quantity discharge position from the origin position;
a starting end inlet port that is provided in the starting-end-side stem internal flow path and is open to the starting end of the stem; and
a peripheral surface discharge port that is provided in the starting-end-side stem internal flow path, is open to an outer peripheral surface of the second intermediate portion of the stem, is positioned in the metering chamber when the stem is arranged at the origin position, moves from the metering chamber to the storage region before the peripheral surface inlet port is positioned in the metering chamber while the stem is moving from the origin position to the metered quantity discharge position, and is positioned in the storage region when the stem is positioned at the metered quantity discharge position.

2. The metering valve according to claim 1, further comprising:
a container-shaped seal member that has a pair of through-holes through which the stem penetrates, forms an inner surface of the internal partition wall, and covers an inner surface of the high-pressure partition wall facing the metering chamber, wherein
the first seal portion and the second seal portion are formed by ends of openings of the pair of through-holes of container-shaped seal member.

3. The metering valve according to claim 2, further comprising:
a cup-shaped fixing member that has a cup shape, is fixed to the high-pressure partition wall so as to cover the container-shaped seal member, fixes the container-shaped seal member, and has a stem fitting portion to which the stem is fitted.

4. The metering valve according to claim 3, wherein
the container-shaped seal member is formed by integrally molding a cup portion that forms the internal partition wall and through which the second seal hole penetrates and a flange portion that covers an opening of the cup portion and through which the first seal hole penetrates so that the second seal hole and the first seal hole are coaxial, and
the cup-shaped fixing member fixes the container-shaped seal member by sandwiching the flange portion of the container-shaped seal member between an end of an opening of the cup-shaped fixing member and the high-pressure partition wall.

5. The metering valve according to claim 3 or 4, further comprising:
a first tubular wall that is provided in the high-pressure partition wall and protrudes outward; and
a first end wall that covers a distal end of the first tubular wall and has a center through which a first fitting hole into which the stem is fitted penetrates, wherein
the cup-shaped fixing member is fitted inside the first tubular wall, and
the container-shaped seal member covers the first end wall and an inner surface of the cup-shaped fixing member.

6. The metering valve according to claim 5, wherein
an inner surface of the first tubular wall of the high-pressure partition wall and an outer peripheral surface of the cup-shaped fixing member have protrusion-recess engagement portions whose protrusion and recess engage with each other.

7. The metering valve according to any one of claims 3 to 6, further comprising:
a fixing member communication hole that is formed in the cup-shaped fixing member and causes inside and outside of the cup-shaped fixing member to communicate with each other; and
a gap that is formed between the cup-shaped fixing member and the container-shaped seal member and allows the high-pressure fluid in the storage region to enter through the fixing member communication hole.

8. The metering valve according to any one of claims 1 to 7, further comprising:
a stem starting-end facing portion that faces the stem from the storage region side in an axial direction of the stem; and
an elastic member that is compressed between the stem starting-end facing portion and the stem to bias the stem to the origin position.

9. The metering valve according to claim 8, wherein
the starting end inlet port is open to an end surface of the stem, and
the elastic member is a compression coil spring, is partially stored in the starting-end-side stem internal flow path, and extends from the starting end inlet port to the stem starting-end facing portion.

10. The metering valve according to claim 9, wherein
a stem receiving cup that overlaps the internal partition wall from the storage region side and is projected in a cup shape toward the storage region, and
a communication hole that penetrates a bottom wall of the stem receiving cup and causes the storage region and the starting-end-side stem internal flow path to communicate with each other are formed, and
the compression coil spring is received by the stem receiving cup and is compressed between the bottom wall of the stem receiving cup and the stem.

11. The metering valve according to any one of claims 1 to 10, further comprising:
a seal path portion that is formed on an outer peripheral surface of the stem, leaves from the second seal portion and is positioned in the metering chamber when the stem is arranged between the origin position and the metered quantity discharge position, and causes the storage region and the metering chamber to communicate with each other over the second seal portion when the stem reaches a continuous discharge position farther away from the origin position than the metered quantity discharge position.

12. The metering valve according to claim 11, wherein
the seal path portion is configured as a recess formed in the outer peripheral surface of the stem.

13. The metering valve according to claim 11 or 12, further comprising:
a mode switching mechanism that switches between a first state in which the stem is allowed to be pressed to the metered quantity discharge position and is restricted not to be further pressed and a second state in which the stem is allowed to be pressed to the continuous discharge position.

14. A high-pressure container, wherein
the high-pressure container is partially formed by the metering valve according to claim 11 or 12 and has the storage region to be filled with asthma medication as the high-pressure fluid.

15. An asthma treatment device including
the high-pressure container according to claim 14, and
a mouthpiece having an L shape as a whole, having a vertical side portion in which a container fitting portion into which the high-pressure container is fitted is provided, having a lateral side portion in which a suction portion to be held in a mouth of an asthma patient is provided, and having an intersection of the vertical side portion and the lateral side portion in which a stem receiving portion receiving the terminal end of the stem is provided, the stem receiving portion being communicating with inside of the suction portion, the asthma treatment device comprising:
a first positioning portion that is provided at a lower end of the stem receiving portion and abuts on a distal end of the stem located at the origin position to position the high-pressure container at a first position of the container fitting portion; and
a second positioning portion that is provided at a lower end of the container fitting portion and, when the high-pressure container is pressed from the first position and the stem abutting on the first positioning portion reaches the metered quantity discharge position, abuts on the high-pressure container to position the high-pressure container at a second position.

16. The asthma treatment device according to claim 15, wherein
a pair of side outside-air suction holes is provided, the pair of side outside-air suction holes being open on both sides of a suction port in the suction portion of the mouthpiece so as to penetrate the mouthpiece and allowing the asthma patient to suck outside air while holding the suction portion in a mouth of the asthma patient and sucking the asthma medication from the suction port.
